Europäisches Patentamt

(19) European Patent Office

Office européen des brevets

(11) Veröffentlichungsnummer: **0 018 569 B1**

(12) # EUROPÄISCHE PATENTSCHRIFT

(45) Veröffentlichungstag der Patentschrift. **21.04.82**

(21) Anmeldenummer: **80102131.2**

(22) Anmeldetag: **21.04.80**

(51) Int. Cl.³: **B 01 J 23/88,** B 01 J 37/02, C 07 C 31/20, C 07 C 29/17

(54) **Verfahren zur Herstellung von Katalysatoren und deren Verwendung zur Hydrierung von Acetylenalkoholen.**

(30) Priorität: **27.04.79 DE 2917018**

(43) Veröffentlichungstag der Anmeldung:
**12.11.80 Patentblatt 80/23**

(45) Bekanntmachung des Hinweises auf die Patenterteilung.
**21.04.82 Patentblatt 82/16**

(84) Benannte Vertragsstaaten:
**BE CH DE FR GB LI NL**

(56) Entgegenhaltungen:
**DE-A-2 231 986**
**DE-A-2 321 101**
**DE-A-2 536 273**
**DE-B-1 285 992**

(73) Patentinhaber: **BASF Aktiengesellschaft,
Carl-Bosch-Strasse 38, D-6700 Ludwigshafen (DE)**

(72) Erfinder: **Baer, Karl, Dr., Kastanienweg 1,
D-6940 Weinheim (DE)**
Erfinder: **Reiss, Wolfgang, Dr., Bannwasserstrasse 70,
D-6700 Ludwigshafen (DE)**
Erfinder: **Schroeder, Wolfgang, Dr., Seebacher
Strasse 51, D-6702 Bad Duerkheim 1 (DE)**
Erfinder: **Voges, Dieter, Dr., Richard-Wagner-Strasse 28,
D-6800 Mannheim 1 (DE)**

## Verfahren zur Herstellung von Katalysatoren und deren Verwendung zur Hydrierung von Acetylenalkoholen

Diese Erfindung betrifft ein Verfahren zur Herstellung von Hydrierkatalysatoren und die Verwendung dieser Katalysatoren für die Hydrierung von Acetylenalkoholen.

Aus der US-A-3 449 445 ist bekannt, daß man 1,4-Butindiol mit guten Ausbeuten zu 1,4-Butandiol hydrieren kann, wenn man Trägerkatalysatoren verwendet, die die Metalle Nickel, Kupfer und Mangan auf Silikagel enthalten. Hydrierungskatalysatoren dieser Art haben nur eine begrenzte Laufzeit, da sich die Kieselsäure im Laufe der Zeit auflöst. Dies hat wiederum die nachteilige Folge, daß sich in Verdampfern und Rohrleitungen beim Dauerbetrieb Siliziumdioxid ablagert, das sich nur auf aufwendige mechanische Weise wieder entfernen läßt.

Bessere Ergebnisse werden mit den in der DE-A-2 536 273 beschriebenen trägerfreien, die Oxide der Metalle Nickel, Kupfer, Molybdän und Mangan enthaltenden Katalysatoren erhalten. Diese Katalysatoren werden durch Fällung einer Lösung der Metallsalze mit Natriumcarbonat, Filtrieren der Carbonatsuspension, Waschen, Trocknen und Tempern hergestellt. Sie zeigen nach Verformung und einer reduktiven Vorbehandlung im technischen Betrieb eine hohe Aktivität und Selektivität, befriedigen aber ebenfalls nicht in der Standzeit. So hat sich beim großtechnischen Dauerbetrieb herausgestellt, daß diese in Strang- oder Tablettenform zum Einsatz gelangenden Oxidgemische nicht formbeständig sind, so daß ein vorzeitiger Zerfall des Katalysators eintritt. Dieser Nachteil läßt sich durch Änderungen der Bedingungen bei der Herstellung des Katalysators nicht vermeiden.

Es bestand deshalb die Aufgabe, für die Hydrierung von Acetylenalkoholen geeignete Katalysatoren zu entwickeln, die neben hoher Aktivität und Selektivität den technisch wichtigen Vorteil einer langen Standzeit aufweisen.

Es wurde nun gefunden, daß bei der Herstellung eines die Oxide der Metalle Nickel, Kupfer, Molybdän und gegebenenfalls Mangan enthaltenden Hydrierungskatalysators durch Fällung einer Lösung von Salzen der Metalle mit Alkalicarbonat, Filtrieren der dabei erhaltenen Suspension, Waschen, Trocknen und Tempern, wobei man das Molybdän vorzugsweise als Ammoniummolybdat vor dem Trocknen zugibt, hinsichtlich der gewünschten Eigenschaften des Katalysators besonders vorteilhafte Ergebnisse erhalten werden, wenn man zu der Metallsalzlösung ein Aluminium- und/oder Eisensalz gibt, die Fällung bei Temperaturen von 35 bis 95°C und einem pH-Wert von 5 bis 9 und das Tempern bei Temperaturen von 350 bis 700°C durchführt, wobei man die Menge der Metallsalze so bemißt, daß die Katalysatormasse nach dem Tempern zu 5 bis 70 Gewichtsprozent aus Aluminium- und/oder Eisenoxid besteht und der Rest neben Nickeloxid als Hauptbestandteil, jeweils bezogen auf Nickeloxid, 20 bis 40 Gewichtsprozent Kupferoxid, 0,5 bis 6 Gewichtsprozent Molybdänoxid und 0 bis 10 Gewichtsprozent Manganoxid enthält.

Das Verfahren zur Herstellung der neuen vorteilhaften Hydrierungskatalysatoren wird z. B. durchgeführt, indem man eine wäßrige Lösung der Metallsalze gleichzeitig und unter Rühren mit einer wäßrigen Alkalicarbonatlösung vermischt, wobei die Metalle in Form eines Carbonat-Hydroxid-Gemisches ausfallen. Als Metallsalze verwendet man z. B. die Nitrate, Sulfate oder Acetate der genannten Metalle, als Alkalicarbonat vorzugsweise Natriumcarbonat.

Die wäßrige Metallsalzlösung hat z. B. einen Metallsalzgehalt von 30 bis 40, vorzugsweise von 34 bis 38 Gewichtsprozent. Die Alkalicarbonatlösung ist z. B. 10 bis 20, vorzugsweise 15 bis 20 gewichtsprozentig. Man fällt bei Temperaturen von 35 bis 95°C, vorzugsweise von 50 bis 90°C und einem pH-Wert von 5 bis 9, vorzugsweise um 7. Die Carbonatsuspension wird nach guter Vermischung filtriert, mit Wasser bis zur Anionenfreiheit gewaschen und bei Temperaturen bis 120°C, z. B. in einem Trockenschrank oder Sprühtrockner getrocknet. Vorzugsweise wird dabei das Molybdän als Ammoniummolybdat dem feuchten Filterkuchen zugegeben.

Die so erhaltene trockene Carbonatmasse wird bei Temperaturen zwischen 350 und 700°C, vorzugsweise zwischen 400 und 600°C getempert. Das Tempern ist beendet, wenn bei der angewendeten Temperatur kein nennenswerter Gewichtsverlust mehr eintritt. Man erhält dabei eine Katalysatormasse, die beim Erhitzen auf 900°C einen Gewichtsverlust (Glühverlust) von 2 bis 10% erleidet und die zu 5 bis 70 Gewichtsprozent, vorzugsweise zu 20 bis 60 Gewichtsprozent aus Aluminium- und/oder Eisenoxid besteht und der Rest neben Nickeloxid als Hauptbestandteil, jeweils bezogen auf Nickeloxid, 20 bis 40, vorzugsweise 28 bis 37 Gewichtsprozent Kupferoxid, 0,5 bis 6, vorzugsweise 1 bis 5 Gewichtsprozent Molybdänoxid und 0 bis 10, vorzugsweise 0 bis 8,5 Gewichtsprozent Manganoxid enthält. Außer diesen Bestandteilen können die Katalysatoren noch geringe Mengen, z. B. 0,05 bis 1,5 Gewichtsprozent andere Metallsalze, wie Natriumoxid enthalten.

Die Katalysatormasse wird vor ihrem Einsatz zweckmäßigerweise konfektioniert, d. h. auf an sich übliche Weise tablettiert oder extrudiert. Beispielsweise preßt man die Katalysatormasse zu Tabletten, indem man sie mit einem Tablettierhilfsmittel, wie Graphit, vermischt und auf einer Tablettenpresse tablettiert. Die Tabletten mit den Dimensionen 4,75 × 4,75 mm haben ein Rüttelgewicht von 900 bis 1200 g/l, eine Porosität (durch Wasseraufnahme bestimmt) von 0,30 bis 0,56 cm$^3$/g und eine Härte von 4000 bis 5000 kg/cm$^2$.

Man kann aus der Katalysatormasse auch Extrudate herstellen, indem man sie mit Wasser verknetet, extrudiert, trocknet und bei 500°C tempert. Im allgemeinen werden hierbei leichtere Katalysatoren erhalten, z. B. solche mit dem Litergewicht: 760 g/l, mit der Porosität: 0,6 cm³/g und der Schneidehärte: 25 N.

Die nach dem neuen Verfahren erhältlichen Katalysatoren werden zweckmäßig vor ihrem Einsatz als Hydrierungskatalysatoren einer an sich üblichen reduzierenden Vorbehandlung unterworfen. Beispielsweise behandelt man den Katalysator zu diesem Zweck etwa 20 bis 40 Stunden bei Temperaturen von 230 bis 280°C.

Die neuen Katalysatoren sind wertvolle Hydrierungskatalysatoren, die insbesondere für die Hydrierung von Acetylenalkoholen, wie Butindiol-1,4, Hexindiol-2,5, 5-Dialkylaminopentin-3-ol-2 und Propargylalkohol hervorragend geeignet sind. Man reduziert die Acetylenalkohole auf an sich übliche Weise an den mit Wasserstoff vorbehandelten Katalysatoren bei Temperaturen zwischen etwa 40 und 180°C und Wasserstoffdrucken von etwa 30 bis 320 bar. Verglichen mit den bisher erhaltenen Ergebnissen bei der Hydrierung von Acetylenalkoholen werden bei der erfindungsgemäßen Verwendung der Katalysatoren höhere Durchsätze bei gleich guter Ausbeute und längere Standzeiten erreicht. Außerdem läßt sich die Reduktion schon bei relativ niedrigen Temperaturen durchführen. Die neuen Katalysatoren weisen auch eine verbesserte Selektivität auf.

Beispiel

a) Herstellung des Katalysators

Eine 20prozentige wäßrige Natriumcarbonatlösung und eine wäßrige Metallnitratlösung, deren Metallnitratgehalte 4,7% NiO, 1,57% CuO, 0,47% Mn₃O₄ und 4,5% Al₂O entsprechen und die eine Dichte von 1,4 g/cm³ aufweist, werden gleichzeitig in ein Rührgefäß in der Nähe des Rührers eingeleitet. Das Rührgefäß ist mit einem Überlauf versehen und hat ein Füllvolumen von 20 l. Die Zudosierung der Metallsalzlösung erfolgt im konstanten Strom in einer Menge von 20 l/h. Dabei wird im Füllgefäß eine Temperatur von 50 bis 95°C, vorzugsweise von 50°C aufrechterhalten und die Sodalösung so dosiert, daß ein mit der Glaselektrode gemessener pH von 7,0 aufrechterhalten wird.

Die überlaufende Carbonatsuspension wird gesammelt und bei 50°C aufbewahrt. Vor der Filtration wird die Suspension in dem Sammelgefäß zur Herstellung einer homogenen Verteilung noch mindestens eine halbe Stunde gerührt. Dabei steigt der pH-Wert auf 7,2 bis 7,5 an. Die Fällung wird filtriert und mit vollentsalztem Wasser gewaschen. Nun wird Ammoniummolybdat in den feuchten Filterkuchen in einer Menge von 2 Gewichtsprozent MoO₃, bezogen auf den Glührückstand, eingearbeitet. Dann wird der Filterkuchen bei 120°C im Trockenschrank oder in einem üblichen Sprühtrockner getrocknet. Man erhält eine Trockenmasse, die die genannten Metalle als ein Hydroxid-Carbonat-Gemisch und außerdem nur noch etwa 0,84% Na₂O und 0,04% NO₃ enthält. Das Hydroxid-Carbonat-Gemisch wird nun 1,5 Stunden bei 500°C getempert, wobei ein Gewichtsverlust von 29% eintritt. Die so erhaltene Katalysatormasse enthält 38,6 Gewichtsprozent NiO, 12,9 Gewichtsprozent CuO, 36,9 Gewichtsprozent Al₂O₃, 3,9 Gewichtsprozent Mn₃O₄, 1,9 Gewichtsprozent MoO₃ und 0,8 Gewichtsprozent Na₂O. Eine Probe der Katalysatormasse erleidet beim Erhitzen auf 900°C einen Glühverlust von 5 Gewichtsprozent.

b) Verwendung des Katalysators für die Hydrierung von 1,4-Butindiol

Das nach Absatz a) erhaltene Katalysatorpulver wird in einem Mischgefäß mit 3% Graphitpulver vermischt und in einer Tablettierpresse tablettiert. Dabei werden Füllungsgrad und Preßdruck so eingestellt, daß das Litergewicht der fertigen Tabletten 1030 Gramm beträgt. Die Porosität der Tabletten beträgt 0,39 cm³/g, der Berstdruck 400 N/cm².

Man füllt in ein druckfestes Reaktionsrohr 0,5 l der Katalysatortabletten ein und behandelt den Katalysator bei 260°C drucklos in einem Stickstoffstrom so mit Wasserstoff, daß die Temperatur 270°C nicht übersteigt. Zum Schluß wird bei 270°C 12 Stunden ein Wasserstoffstrom über den Katalysator geleitet. Dann wird der Katalysator auf 30°C abgekühlt und mit Wasser benetzt. Man leitet nun eine 8,2prozentige wäßrige Lösung von 1,4-Butindiol, die außerdem 38,8% Butandiol, 0,2% Formaldehyd und 0,3% Butanol enthält, über den Katalysator. Dabei betragen die Katalysatorbelastung 0,46 kg Butindiol/l Katalysator und h, die Flüssigkeitsbelastung 40 m³/m² und h und die Gasbelastung 30 m³/m² und h. Die Reaktionstemperatur beträgt 140°C, der Druck 260 bar. Man erhält Butandiol in einer Ausbeute von 98,5%.

**Patentansprüche**

1. Verfahren zur Herstellung eines die Oxide der Metalle Nickel, Kupfer, Molybdän und gegebenenfalls Mangan enthaltenden Hydrierungskatalysators durch Fällung einer Lösung von Salzen der Metalle mit Alkalicarbonat, Filtrieren der dabei erhaltenen Suspension, Waschen, Trocknen und Tempern, wobei man das Molybdän vorzugsweise als Ammoniummolybdat vor dem Trocknen zugibt, dadurch gekennzeichnet, daß man zu der Metallsalzlösung ein Aluminium- und/oder Eisensalz gibt, die Fällung bei Temperaturen von 35 bis 95°C und einem pH-Wert von 5 bis 9 und das Tempern bei Temperaturen von 350 bis 700°C durchführt, wobei man die Menge der Metallsalze so bemißt, daß die Katalysatormasse nach dem Tempern zu 5 bis 70 Gewichtsprozent aus Aluminium- und/oder Eisenoxid besteht und der Rest neben Nickeloxid als Hauptbestandteil, jeweils bezogen auf Nickeloxid, 20 bis 40 Gewichtsprozent Kupferoxid, 0,5 bis 6 Gewichtsprozent Molybdänoxid und 0 bis 10 Gewichtsprozent Manganoxid enthält.

2. Verfahren nach Anspruch 1, dadurch gekennzeichnet, daß die Katalysatormasse nach dem Tempern zu 20 bis 60 Gewichtsprozent aus Aluminium- und/oder Eisenoxid besteht und der Rest neben Nickeloxid als Hauptbestandteil, jeweils bezogen auf Nickeloxid, 28 bis 37 Gewichtsprozent Kupferoxid, 1 bis 5 Gewichtsprozent Molybdänoxid und 0 bis 8,5 Gewichtsprozent Manganoxid enthält.

3. Verwendung der nach den Ansprüchen 1 und 2 erhaltenen Katalysatoren für die Hydrierung von Acetylenalkoholen.

## Claims

1. A process for the preparation of a hydrogenation catalyst containing the oxides of the metals nickel, copper and Molybdenum, with or without manganese, by precipitating a solution of salts of the metals with an alkali metal carbonate, filtering the resulting suspension and washing, drying and heating the filter residue, the molybdenum being preferably added as ammonium molybdate prior to drying, characterized in that an aluminium salt and/or iron salt is added to the metal salt solution, the precipitation is carried out at from 35 to 95°C and a pH of from 5 to 9 and the heating is carried out at from 350 to 700°C, the amount of metal salts being so chosen that, after heating, the catalyst consists of from 5 to 70 per cent by weight of aluminium oxide and/or iron oxide, and the remainder containing, apart from nickel oxide as the main component, from 20 to 40 per cent by weight of copper oxide, from 0.5 to 6 per cent by weight of molybdenum oxide and from 0 to 10 per cent by weight of manganese oxide, in each case based on nickel oxide.

2. A process as claimed in claim 1, characterized in that the catalyst, after heating, consists of from 20 to 60 per cent by weight of aluminium oxide and/or iron oxide, and the remainder contains, apart from nickel oxide as the main component, from 28 to 37 per cent by weight of copper oxide, from 1 to 5 per cent by weight of molybdenum oxide and from 0 to 8.5 per cent by weight of manganese oxide, in each case based on nickel oxide.

3. Use of a catalyst obtained al clamed in claims 1 and 2 for the hydrogenation of acetylene-alcohols.

## Revendications

1. Procédé de préparation d'un catalyseur d'hydrogénation, contenant les oxydes des métaux nickel, cuivre et molybdène et éventuellement du manganèse, par précipitation des sels de ces métaux en solution par un carbonate de métal alcalin, filtration de la suspension formée, lavage et séchage, suivi d'un traitement thermique, le molybdène étant de préférence ajouté avant le séchage sous forme de molybdate d'ammonium, caractérisé en ce que l'on ajoute à la solution des sels de métaux un sel d'aluminium et(ou) de fer, on effectue la précipitation à des températures de 35 à 95°C et à un pH de 5 à 9 et le traitement thermique entre 350 et 700°C, les proportions respectives des sels de métaux étant choisies telles que la matière à activité catalytique, ayant subi le traitement thermique, est composée de 5 à 70% en poids d'oxyde d'aluminium et(ou) d'oxyde de fer, le restant étant constitué d'oxyde de nickel comme composant principal, ainsi que, par rapport à l'oxyde de nickel, de 20 à 40% en poids d'oxyde de cuivre, de 0,5 à 6% en poids d'oxyde de molybdène et de 0 à 10% en poids d'oxyde de manganèse.

2. Procédé suivant la revendication 1, caractérisé en ce que la matière à acitivité catalytique est composée, après le traitement thermique, de 20 à 60% en poids d'oxyde d'aluminium et(ou) d'oxyde de fer, le restant étant constitué d'oxyde de nickel comme composant principal, ainsi que, par rapport à l'oxyde de nickel, de 28 à 37% en poids d'oxyde de cuivre, de 1 à 5% en poids d'oxyde de molybdène et de 0 à 8,5% en poids d'oxyde de manganèse.

3. Utilisation d'un catalyseur préparé par le procédé suivant l'une des revendications 1 et 2 pour l'hydrogénation d'alcools acétyléniques.